# EUROPEAN PATENT APPLICATION

(11) **EP 4 779 308 A1**
(43) Date of publication of application: **22.07.2026**
(21) Application number: 25152872.5
(22) Date of filing: 20.01.2025
(51) Int. Cl.: G01N 33/543

(54) **DETECTION OF BIOMARKERS IN A SAMPLE**

(71) Applicant: QU-IP B.V., 1015 CS Amsterdam (NL)
(72) Inventor: VAN DER SCHEE, Marc Philippe, 1015 CS Amsterdam (NL); APHRHAM, Samer Gerges, 1015 CS Amsterdam (NL); KOVACS LENDVAI, Agnes, 1015 CS Amsterdam (NL); VERHEIJDEN, Mark Lloyd, 1015 CS Amsterdam (NL)
(74) Representative: Arnold & Siedsma

(57) **Abstract**

The present invention relates to a method for detecting a biomarker in a sample, comprising the steps of a) contacting a sample that potentially comprises a biomarker with optically responsive magnetic particles capable of binding the biomarker, wherein presence of a biomarker results in the generation of an optical response; b) concentrating the particles by means of magnetic force; c) resuspending the particles in a fluid; and d) detecting in the fluid whether an optical response is generated. The invention further relates to a kit of parts for performing the method and to particles for use in the method, which particles comprise optically responsive magnetic particles coated with a noble metal and/or decorated with one or more receptor molecules.

## Description

The present invention relates to a method for detecting a biomarker in a sample. The invention further relates to a kit of parts for performing the method and to particles for use in the method.

Biomarkers are measurable indicators found in the body that can signal a wide range of physiological and pathological processes. Biomarkers are often disease-related reporter molecules and are used in medical research and healthcare to detect, diagnose, and monitor diseases. Molecular biomarkers include DNA, RNA, peptides, proteins, and metabolites. They can indicate genetic mutations, protein levels, or metabolic changes associated with diseases. There are different types of markers, in particular endogenous and exogenous biomarkers. Endogenous biomarkers are produced by the body naturally. Exogenous biomarkers are markers that result for example from the interaction of administrated reagents with the body.

Biomarkers can help identify diseases at an early stage, often before symptoms appear. This early detection can significantly improve treatment outcomes and survival rates, especially for conditions like cancer. Furthermore, biomarkers can provide valuable information for diagnosing diseases and monitoring their progression. They may help in understanding how a disease is evolving and whether treatments are effective.

It is desirable for medical testing to be performed in a point-of-care (PoC) situation, i.e. near the site of patient care, rather than in a centralized laboratory. The goal of point-of-care testing and treatment is to provide immediate results and interventions, which can lead to faster decision-making and improved patient outcomes. PoC testing reduces the logistical and financial barriers associated with traditional testing, such as travel time and costs, thereby facilitating increased access and expanding the potential reach to a broader target population.

It is the object of the present invention to provide a novel method for the direct, point-of-care (PoC) detection and reporting of biomarkers, characterized by straightforward and easily interpretable signal generation, thus enhancing user accessibility and diagnostic efficiency.

In the research leading to the present invention, a combination in one system was developed, comprising a 2-step approach of first concentration enlargement of biomarkers present in a sample of body fluid or tissue and second, the detection or visualization of the biomarkers. The system was developed for nucleic acids including chemically modified nucleic acids, but can also be used for other biomolecules.

The invention thus relates to a method for detecting a biomarker in a sample, comprising the steps of:
a) contacting a sample that potentially comprises a biomarker with optically responsive magnetic particles capable of binding the biomarker, wherein presence of a biomarker results in the generation of an optical response;
b) concentrating the particles by means of magnetic force;
c) resuspending the particles in a fluid; and
d) detecting in the fluid whether an optical response is generated

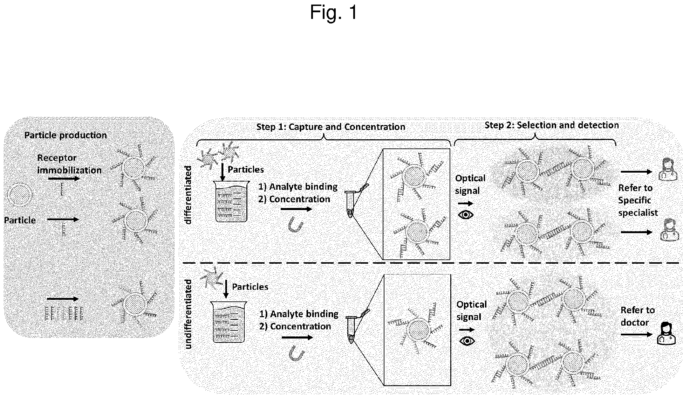

The method according to the invention is schematically shown in **Figure 1****.**

In one embodiment, the optical response is generated by binding of the biomarker to the particle or by activating the collateral cleavage activity of a CRISPR-Cas enzyme. The optical signal generated in the fluid is a change in the absorption and emission of electromagnetic radiation, in particular a colour change. Typically, the (colour) change is the result of either clustering of the particles in the fluid or the unclustering of particle aggregates, triggered by binding of the biomarker. Alternatively, the (colour) change can also be the result of binding of the biomarker to the particles.

The optical changes are either colour changes that are visible by the naked human eye, or changes in electromagnetic radiation that are invisible to the eye but can be observed by optical detectors. Optical detectors or photo detectors are sensors that convert electromagnetic radiation into electric signals that can be measured through an appropriate device. An example of an optical detector is the photodiode. The output electric signal thus obtained is proportional to the incident light or electromagnetic radiation. Optical detectors can be used to detect both visible and invisible electromagnetic radiation.

For the optical signal to occur, the particles are functionalized with a layer that interacts with the biomarker (also called analyte). This layer can for example consist of immobilized receptor molecules such as nucleic acid or derivatives thereof or peptide receptors that are able to specifically bind DNA, RNA or proteins and peptides. The specific binding is the result of hybridization of the nucleic acid biomarker with a complementary nucleic acid receptor molecule or ligand-receptor interaction in the case of peptides and proteins.

The particles may be decorated with one type of receptor molecule with one specificity or with two, three, four or more different types of receptor molecule with each a separate specificity. Multiple particle batches each with other receptor molecules and various optically responsive particle types, each with a different optical characteristic to connect various colours with various biomarkers result in a differentiated readout (*i.e.* multiplexing).

In a further embodiment, one particle batch can be functionalized with multiple receptor molecules on each particle and result in a yes/no (colour) change for the presence of any of the analyte molecules. This embodiment results in an undifferentiated readout.

Alternatively or in addition, the particles are coated with a transition metal, and in particular a noble metal, more in particular selected from gold, silver, platinum, palladium, rhodium, ruthenium, osmium and iridium for binding the biomarker. In a preferred embodiment, the transition metal is a soft metal. In a more preferred embodiment, the soft metal is gold.

Thiols, dialkylsulfides, dialkyldisulfides and phosphorothioate are known to be chemisorbed with high affinity on gold. Therefore, DNA that is chemically modified with these sulphur-bearing groups can directly bind to a gold surface without the need for a receptor molecule. In a particular embodiment, the interaction between the particle and sulphur-containing biomarkers is thus directly with the surface of a gold layer.

In a further embodiment, the sulphur-containing biomarker is phosphorothioate DNA. Phosphorothioate DNA is DNA in which a nonbridging oxygen is replaced by a sulphur atom. Binding of phosphorothioate DNA is non-sequence specific.

Gold-coated particle clustering results in a detectable colour change. This binding is aspecific to the sequence . and multiplexing will be limited to the level of detecting the presence of any of the target biomarkers but not specifically which one. Thus, this type of assay can detect multiple biomarkers but cannot distinguish between them.

The method of the invention is also very useful in the detection of DNA barcodes that are used in the PATROL platform (Zhong Q, Tan EKW, Martin-Alonso C, Parisi T, Hao L, Kirkpatrick JD, Fadel T, Fleming HE, Jacks T, Bhatia SN. Inhalable point-of-care urinary diagnostic platform. Sci Adv. 2024 Jan 5;10(1):eadj9591. doi: 10.1126/sciadv.adj9591. Epub 2024 Jan 5. PMID: 38181080; PMCID: PMC10776015). The PATROL platform formulates a set of DNA-barcoded peptides into an inhalable format. Each peptide is specifically cleaved by a particular protease that is representative for a particular form of cancer. Upon cleavage, the associated DNA-barcode is released and excreted via the urine. The DNA-barcode detected in a urine sample is thus diagnostic for the cancer that is present in the patient. The method of the invention is particularly useful to detect these DNA barcodes.

The particles are also magnetic, which allows the pull-down of particles with bound analytes from (sometimes large) liquid biopsy volumes. Reconstitution of the particles that are separated from the sample in a clean, controlled and much smaller volume, effectively results in analyte concentration increase.

The receptor molecules with which the magnetic particles are decorated can be nucleic acids, nucleic acid derivatives, peptides, proteins.

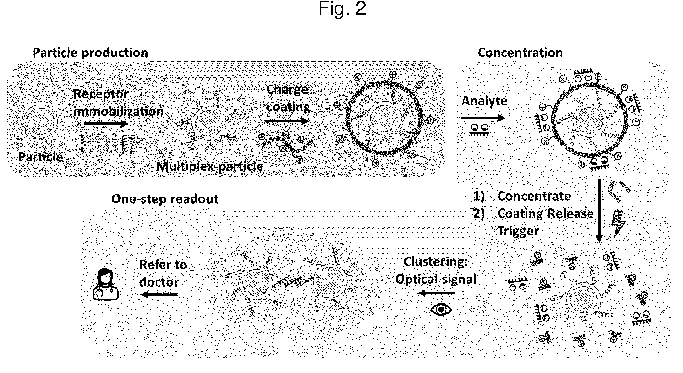

In a further embodiment, the magnetic particles are decorated with a receptor molecule capable of binding the biomarker and subsequently coated with a charge coating. The method comprises the further step of releasing the charge coating between steps c) and d). Such two-layer coating enables the stepwise capture of the biomarker using electrostatic interactions between the DNA/RNA and the positive charge, followed by triggered charge removal such as enzymatic degradation of the charge coating. This is schematically shown in **Figure 2****.**

The advantage of the additional charge coating is that all targets will bind to all beads, requiring a total of less beads in the (large) sample to achieve target binding at a similar rate. If multiple particle batches without the charge coating are used to allow multiplexing, each batch will need to be used at a similarly high concentration as the double layer ensemble batch to achieve similar binding rate of the specific target, requiring the total combined bead loading to be much higher. In addition, the denaturation of dsDNA targets in the case of large (urine) samples is impractical, complicating the detection of double stranded DNA (dsDNA) in this situation. By addition of the charge coating, all DNA is strongly concentrated before the specific hybridization to the receptors on the particles. This makes denaturation of dsDNA much more feasible.

In a further embodiment, the particles are quantum dots (QD). Quantum dot clustering results in colour change. Using magnetic QDs facilitates magnetic precipitation. In order to detect and indicate each multiplex biomarker, various colours QDs can be combined in one-pot and the outcome colour can indicate the specific biomarker(s) present in the sample.

In one embodiment, the surface charge on the particle is in the form of a strong ion exchange coating, such as a coating bearing quaternary ammonium ions, to facilitate binding of short DNA or DNA-derivative oligonucleotides even in high-salt media such as urine.

In one embodiment, the detection is phone-camera aided. Using a phone-camera for detection minimizes observer-bias and facilitates read-out for visually impaired people, in particular colour blind people. Alternatively, the change is observed with a spectroscope or another device that can detect changes in visible and invisible electromagnetic radiation.

The method of the invention may further comprise a step for amplifying the optical response generated in a positive feed-back loop, in particular by means of a cascade reaction, or by means of an autocatalytic process, or by means of a collateral cleavage effect.

Sensitivity increase is then obtained by presentation of the receptor molecules in such a manner that binding of the biomarker results in a positive feedback loop. This can subsequently enable (un)clustering of particles or results in the generation of a fluorescence signal making use of the Forster Resonance Energy Transfer (FRET) effect to generate or quench fluorescence.

The feedback loop can be generated by a cascade reaction where the binding of one biomarker changes the receptor molecule in such a way that neighboring receptor molecules also change. An example of such feedback loop is the hybridization chain reaction (HCR; Evanko, D. Hybridization chain reaction. Nat Methods 1, 186 (2004)).

In another embodiment, increase of the sensitivity of the assay is achieved by an autocatalytic process where the binding of the biomarker releases an agent that also bind to the receptor molecule. An example is the technology described in "RaPID Platform for the Discovery of Pseudo-Natural Macrocyclic Peptides" (Yuki Goto and Hiroaki Suga; Accounts of Chemical Research 54 (18), 3604-3617 (2021)) where the target DNA gets translated multiple times into various peptides allowing signal amplification and multiplexing.

In one embodiment, the optical response is detected by means of a particle-based lateral flow assay. These assays are typically more sensitive than colour change from gold particle clustering. Multiple test-spots can facilitate the detection and identification of each multiplex biomarker.

The readout can also be done by using pre-clustered particles (like gold or QD) that will be released and change in colour as an effect of collateral cleavage activity of CRISPR-Cas12, activated in presence of the biomarker. The collateral cleavage effect can increase overall sensitivity. In order to detect and indicate each multiplex biomarker, the sample would need to be split in as many aliquots. This can be combined with autocatalytic sensitivity increase by using a system such as "CRISPR-Cas-only amplification network (CONAN) (K. Shi, S. Xie, R. Tian, S. Wang, Q. Lu, D. Gao, C. Lei, H. Zhu and Z. Nie, Sci. Adv., 2021, 7, eabc7802). In this platform, recognition of the DNA biomarker activates transducer 2, which comprises Cas12a and a dsDNA probe, and the resulting Cas12a trans-cleavage activity liberates a caged crRNA that can then target transducer 2, creating a positive feedback circuit that generates an exponentially increasing fluorescent signal.

The invention further relates to a kit of parts for performing the method, comprising optically responsive magnetic particles coated with a noble metal and/or decorated with one or more receptor molecules and means for detecting an optical signal generated by the particles after binding of a biomarker.

Also part of the invention are particles for use in the method, comprising optically responsive magnetic particles coated with a noble metal and/or decorated with one or more receptor molecules.

The present invention will be further elucidated in the examples that follow and that are given for illustration purposes only and are not intended to limit the invention in any way.

### EXAMPLES

### EXAMPLE 1

### Differentiated target binding

In this example, the particles are coated with a DNA receptor layer that directly and specifically bind the ssDNA target from the sample as schematically shown in Figure 1 and specified in that figure as "differentiated".

Nanoparticles ranging from 15 to 100 nm in diameter with a magnetic core and gold shell ranging in thickness between 3 to 40 nm thickness were synthesized according to literature procedures or alternatively are commercially available, for example from the company CD Bioparticles (https://www.cd-bioparticles.com/*)*.

For each target DNA, a set of two receptor functionalized particles are produced. One particle batch binds one half of the target DNA molecule specifically. The other particle batch binds the other half of that same DNA molecule specifically. As a result, the DNA target can bind one particle from each batch together, also called "DNA handcuffing".

These particles are functionalized with DNA probes using one of the following methods.

### Method 1

Magnetic gold nanoparticles (MAuNPs) are transferred in PBS buffer, pH 7.4. An excess of thiolated DNA probes is added to a final concentration of 1uM in the nanoparticle solution. The reaction mixture is mixed by vortexing and incubated at room temperature for at least 1h. NaCl solution is added gradually to the mixture to reach a final concentration of 0.3 M. The mixture is incubated at room temperature overnight. The MAuNPs are pelleted using a magnet and the supernatant containing unbound DNA is removed and replaced with PBS. The washing with PBS is repeated 3 more times . The DNA-conjugated nanoparticles are stored at 4 °C.

### Method 2

Poly-L-Lysine (PLL) with 5% of the lysine residues functionalized with Strained Alkyne groups (PLL-SA) such as dibenzocyclooctyne (DBCO) or bicyclononyne (BCN) are synthesized according to literature protocol (Langmuir 2020, 36, 16, 4272-4279 (https://doi.org/10.1021/acs.langmuir.0c00144)) or purchased from specialized vendor such as Susos (susos.com).

The MAuNPs are transferred in PBS buffer, pH 7.4. An excess PLL-SA is added to a final concentration of 1 mg/mL in the nanoparticle solution. The mixture is incubated for 1 h on roller-shaker. The MAuNPs are magnetically pelleted and excess PLL-SA is replaced with PBS. The washing is repeated 3 more times. Excess azide functional DNA probes are added to a final concentration of 1uM in the nanoparticle solution. The mixture is incubated at room temperature for 2 h. The MAuNPs are pelleted using a magnet and the supernatant containing unbound DNA removed and replaced with PBS. The washing is repeated 3 more times. The DNA-conjugated nanoparticles are stored at 4°C.

MAuNPs display different optical properties by tuning the size of the particle from 15 to 100nm and thickness of the gold layer in the range of 3 to 40 nm. For each target biomarker, a different combination of two particles is selected and decorated with DNA probes as specified above. The various nanoparticle batches are mixed to allow multiplexing in one-pot.

The nanoparticle mixture is added to the sample, e.g. urine. The sample can contain no, one or multiple single stranded DNA (ssDNA) reporters.

After incubation for 1h the beads are magnetically collected and the supernatant removed. The beads are resuspended in 100uL of 4xSSC buffer at pH 7.4 and allowed to further interact for 15 min. This concentrated formulation will allow further development of the "DNA handcuffing" process but is also needed for effective optical readout.

The final color is compared to a separate particle batch of the same original batch that had not been in contact with the sample for reference.

The signal can be read out using a phone camera and deconvoluted to determine which nanoparticles are clustered and thus which reporters were present in the original sample. The diagnostic result can be sent digitally to a healthcare professional.

### EXAMPLE 2

### Particles with charge coating

In this example, the particles are coated with 2 layers. An inner receptor layer and an outer PLL layer. All DNA is first captured aspecifically by the PLL layer after which the PLL layer is cleaved enzymatically and the DNA biomarkers bind specifically to the DNA receptors in the inner layer.

MAuNPs are functionalized with DNA probes according to method 1 of example 1, so using the thiolated DNA method only. DNA-functionalized MAuNPs are then incubated with 1mg/mL of PLL in PBS for 2h on a roller-shaker. Excess PLL is washed 3x using magnetic precipitation of particles.

This 2-layer MAuNPs is then added to the sample, e.g. urine. The sample can contain no, one or multiple ssDNA reporters or double stranded DNA (dsDNA) targets. All ssDNA or dsDNA now binds to the outer PLL coating by charge-interaction.

The MAuNPs are pelleted magnetically and washed 3x with PBS. 100uL of 2.5mg/mL trypsin solution in HEPES saline buffer at pH 7.4 is added to the cleaned bead pellet and incubated for 1h at room temperature on roller shaker. During this process, the outer PLL layer is enzymatically broken down and the DNA released.

Now there are 2 options depending on the presence of ssDNA or dsDNA targets
ssDNA will bind in a sequence specific manner to the inner DNA receptor layer, resulting in MAuNPs clustering and subsequential color change in presence of the specific biomarker(s)

For dsDNA targets the now small sample volume is heated to 95°C to denature the dsDNA after which the sample is cooled down and the now ssDNA target bind in a sequence specific manner to the inner DNA receptor layer, resulting in MAuNPs clustering and subsequential color change.

The final color is compared to a separate batch of the same particles that had not been in contact with the sample for reference. The signal can be read out using a phone camera and deconvoluted to determine which nanoparticles are clustered and thus which reporters were present in the original sample. The diagnostic result can be sent digitally to a healthcare professional.

### EXAMPLE 3

### Method with clustered particles

In this example, particles are pre-clustered in small clusters and the un-clustering will give the optical signal. Particles are clustered by making 2 batches or MAuNPs. One with DNA probe "A" and one with DNA probe "B". A third and free dissolved synthetic oligo complementary to both "A" and "B" is used to 'handcuff' the particles together. This third oligo "C" additionally has a short single stranded section after binding to "A" and "B" that is susceptible to the collateral cleavage activity of CRISPR-Cas12 proteins, which will result in un-clustering and consequential color change.

MAuNPs are functionalized with DNA according to method 1 of example 1, so using the thiolated DNA method only. However, only 2 DNA receptors are used now ("A" and "B") rather than one set of two probes for each specific target biomarker. So a final total of 2 batches of functionalized MAuNPs is created, independent on the amount to different targets.

The MAuNPs batch with receptor "A" is incubated with 1uM of oligo "C" and allowed to hybridize for 1h. The MAuNPs are pelleted magnetically and washed 3x with PBS. Now, under vigorous stirring, the MAuNPs batch with receptor "B" is very slowly titrated to these particles to a final 1:1 molar ratio of the "A" and "B" particles. The now clustered particles are coated with the 2^{nd} PLL layer as described in Example 2.

DNA from the sample is captured, concentrated and released as in in Example 2. CRISPR-Cas12 loading with the RNA guide gives a Ribonucleoprotein (RNP) Complex specific for the dsDNA target complementary to the RNA guide. RNP complexes can be commercially purchased. For each target sequence, a complementary RNA guide for CRISPR-Cas12 is be designed to allow multiplexing.

## Claims

1. Method for detecting a biomarker in a sample, comprising the steps of:
a) contacting a sample that potentially comprises a biomarker with optically responsive magnetic particles capable of binding the biomarker, wherein presence of a biomarker results in the generation of an optical response;
b) concentrating the particles by means of magnetic force;
c) resuspending the particles in a fluid; and
d) detecting in the fluid whether an optical response is generated.

2. Method as claimed in claim 1, wherein the optical response is generated by binding of the biomarker to the particle or by activating the collateral cleavage activity of a CRISPR-Cas enzyme.

3. Method as claimed in claim 1 or 2, wherein the particles are decorated with a receptor molecule capable of binding the biomarker, in particular specifically binding the biomarker.

4. Method as claimed in claim 3, wherein the receptor molecule is selected from nucleic acids, nucleic acid derivatives, peptides, proteins.

5. Method as claimed in any one of the claims 1 to 4, wherein the particles are coated with a transition metal, in particular a noble metal selected from gold, silver, platinum, palladium, rhodium, ruthenium, osmium and iridium for binding the biomarker and/or the receptor molecule.

6. Method as claimed in claim 5, wherein the particles are coated with gold for binding a biomarker comprising a sulphur-bearing group, such as phosphorothioate DNA.

7. Method as claimed in any one of the claims 1 to 6, wherein the optical response is a colour change resulting from binding of the biomarker to the particles, clustering of the particles or unclustering of the particles.

8. Method as claimed in claim 7, wherein the colour change is visible for the naked eye or in the invisible spectrum.

9. Method as claimed in any one of the claims 1 to 8, wherein the particles are decorated with a receptor molecule capable of binding the biomarker and coated with a charge coating and the method between steps c) and d) comprises the further step of releasing the charge coating.

10. Method as claimed in any one of the claims 1 to 9, wherein the particles are quantum dots.

11. Method as claimed in any one of the claims 1 to 10, wherein the particles are decorated with one type of receptor molecule with one specificity or wherein the particles are decorated with two or more different types of receptor molecule with each a separate specificity.

12. Method as claimed in any one of the claims 1 to 11, further comprising a step for amplifying the optical response generated in a positive feed-back loop, in particular by means of a cascade reaction such as the hybridization chain reaction (HCR), or by means of an autocatalytic process, or by means of a collateral cleavage effect.

13. Method as claimed in any one of the claims 1 to 12, wherein the optical response is detected by means of a particle-based lateral flow assay, a spectroscope, or a smartphone.

14. Kit of parts for performing the method as claimed in any one of the claims 1 to 13, comprising optically responsive magnetic particles coated with a noble metal and/or decorated with one or more receptor molecules and means for detecting an optical signal generated by the particles after binding of a biomarker.

15. Particles for use in the method as claimed in any one of the claims 1 to 13, comprising optically responsive magnetic particles coated with a noble metal and/or decorated with one or more receptor molecules.
